(19) Europäisches Patentamt
European Patent Office
Office européen des brevets

(11) **EP 3 556 293 A1**

(12) **EUROPÄISCHE PATENTANMELDUNG**

(43) Veröffentlichungstag:
**23.10.2019 Patentblatt 2019/43**

(51) Int Cl.:
***A61B 6/00*** *(2006.01)*

(21) Anmeldenummer: **18168142.0**

(22) Anmeldetag: **19.04.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH 91052 Erlangen (DE)**

(72) Erfinder: **Hofmann, Christian 91052 Erlangen (DE)**

Bemerkungen:
Geänderte Patentansprüche gemäss Regel 137(2) EPÜ.

(54) **VERFAHREN ZUR BILDERZEUGUNG BEI EINEM RÖNTGENGERÄT, BILDERZEUGUNGSRECHNER, RÖNTGENGERÄT, COMPUTERPROGRAMMPRODUKT UND COMPUTERLESBARER DATENTRÄGER**

(57) Bei dem erfindungsgemäßen Verfahren zur Bilderzeugung bei einem Röntgengerät (1), werden Röntgenbilddaten (I0,P0) bereitgestellt und aus den Röntgenbilddaten (I0,P0) Hochfrequenzbilddaten (P0HF), die eine Hochfrequenzinformation enthalten, extrahiert. Die extrahierten Hochfrequenzbilddaten (P0HF) werden dann einer nichtlinearen Korrektur unterworfen, und mittels der korrigierten Hochfrequenzbilddaten (P0HFS,P0HFSW) korrigierte Bilddaten (IHFS) erstellt.

FIG 3

EP 3 556 293 A1

**Beschreibung**

Die Erfindung betrifft ein Verfahren zur Bilderzeugung bei einem Röntgengerät. Des Weiteren betrifft die Erfindung einen Bilderzeugungsrechner, der insbesondere zur Durchführung des Verfahrens eingerichtet ist, sowie ein Röntgengerät, insbesondere mit einem solchen Bilderzeugungsrechner. Außerdem betrifft die Erfindung ein Computerprogrammprodukt und einen computerlesbaren Datenträger, der das Computerprogrammprodukt umfasst.

Röntgengeräte werden häufig - beispielsweise in Form eines Computertomographiegeräts - zur medizinischen Bildgebung für Untersuchungen eingesetzt. Die Röntgengeräte umfassen dabei üblicherweise eine Röntgenstrahlenquelle (kurz: "Röntgenquelle") und einen zu dieser in Gegenüberstellung angeordneten Röntgenstrahlen-Detektor (kurz: "Detektor"). Zur Bilderzeugung wird das zu untersuchende Objekt - d. h. üblicherweise der Patient oder zumindest ein Körperteil des Patienten - in den Strahlengang zwischen der Röntgenquelle und dem Detektor eingebracht. Der Detektor ist dazu eingerichtet, die von dem Objekt bei der Durchleuchtung teilweise abgeschwächte Strahlung zu erfassen. Aus den von dem Detektor ausgegebenen Mess-Signalen wird dabei eine Intensitätsverteilung der auftreffenden Röntgenstrahlung, üblicherweise in Form von Graustufen (oder: Grauwerten) abgeleitet. Bei einem Computertomographiegerät wird das aus der Röntgenquelle und dem Detektor gebildete Röntgensystem um das Objekt rotiert und aus der dabei aufgenommenen Vielzahl an Einzel-Rohbildern, die üblicherweise jeweils einen linienförmigen Intensitätsverlauf darstellen, ein in der Strahlungsebene zwischen der Röntgenquelle und dem Detektor liegendes Schnittbild rekonstruiert.

Für den Fall, dass das Objekt, konkret der Patient metallische Teile, beispielsweise Implantate, eine Zahnspange oder dergleichen enthält, kommt es häufig zu sogenannten "Metall-Artefakten". Diese äußern sich beispielsweise als sogenannte Hochfrequenz-Artefakte, die aufgrund der begrenzten räumlichen Auflösung des Detektors und vergleichsweise "scharfen" Kanten von Strukturen mit hoher Röntgenstrahlungsabsorption (bspw. ein Metallteil) gerade im rekonstruierten Schnittbild häufig zu strahlenartigen Streifen oder "falschen" Kanten führen, die interessante Konturen überdecken können, und somit eine Auswertung des Schnittbilds erschweren oder gar verhindern können.

Aus der Druckschrift DE 10 2016 202 434 A1 ist ein Verfahren zur Auswahl eines Algorithmus zur Korrektur wenigstens eines Bildartefaktes bekannt, wobei das Verfahren folgende Schritte umfasst: Identifizieren anhand des Bilddatensatzes wenigstens eines den Bildartefakt verursachenden Objektbestandteils innerhalb der interessierenden Region des Untersuchungsobjektes, Ermitteln anhand des Bilddatensatzes wenigstens einer den Objektbestandteil beschreibenden Kenngröße, Ermitteln eines Artefaktkorrekturalgorithmus basierend auf der wenigstens einen Kenngröße, und Anwenden des Artefaktkorrekturalgorithmus auf den Bilddatensatz.

Aus der Druckschrift DE 10 2014 217 966 A1 ist ein Verfahren zur Reduktion von Metallartefakten in CT-Bilddatensätzen umfassend die folgenden Verfahrensschritte: Anwendung von N > 1 unterschiedlichen Metallartefakt-Reduktionsalgorithmen auf CT-Rohdaten und/oder Anwendung von Metallartefakt-Reduktionsalgorithmen der gleichen Art mit unterschiedlichen Parametern, Erzeugung von korrigierten Rohdatensätzen, Bestimmung eines datenpunktweisen Mischungsgewichts für die CT-Rohdaten, sowie Bestimmung von datenpunktweisen Mischungsgewichten für die korrigierten Rohdatensätze in einer Kontrolleinheit, datenpunktweise Mischung der Rohdatensätze und korrigierten Rohdatensätze, und CT-Rekonstruktion der gemischten Daten zu einem finalen CT-Bild.

Der Erfindung liegt die Aufgabe zugrunde, eine Verbesserung der Bildqualität von Röntgenbildern zu ermöglichen.

Diese Aufgabe wird erfindungsgemäß gelöst durch ein Verfahren zur Bilderzeugung mit den Merkmalen des Anspruchs 1. Des Weiteren wird diese Aufgabe erfindungsgemäß gelöst durch einen Bilderzeugungsrechner mit den Merkmalen des Anspruchs 11. Weiterhin wird diese Aufgabe erfindungsgemäß gelöst durch ein Röntgengerät mit den Merkmalen des Anspruchs 12. Außerdem wird diese Aufgabe erfindungsgemäß gelöst durch ein Computerprogrammprodukt mit den Merkmalen des Anspruchs 13 sowie durch einen computerlesbaren Datenträger mit den Merkmalen des Anspruchs 14. Weitere vorteilhafte und teils für sich erfinderische Ausführungsformen und Weiterbildungen der Erfindung sind in den Unteransprüchen und der nachfolgenden Beschreibung dargelegt.

Das erfindungsgemäße Verfahren dient zur Bilderzeugung bei einem Röntgengerät, vorzugsweise also zur Bilderzeugung aus den mittels des Röntgengeräts gesammelten Durchleuchtungs-Daten. Verfahrensgemäß werden dabei zunächst Röntgenbilddaten bereitgestellt. Aus diesen Röntgenbilddaten werden Hochfrequenzbilddaten, die eine Hochfrequenzinformation (oder auch: einen Hochfrequenzinhalt der Röntgenbilddaten) enthalten, extrahiert. Die extrahierten Hochfrequenzbilddaten werden anschließend einer nichtlinearen Korrektur unterworfen. Des Weiteren werden mittels der korrigierten Hochfrequenzbilddaten korrigierte Bilddaten erstellt.

Der Hochfrequenzinhalt (und somit auch die Hochfrequenzinformation) eines (Röntgen-)Bildes stellt hierbei insbesondere auch ein Maß für einen Detailreichtum, insbesondere für die Größe der in den Röntgenbilddaten abgebildeten, insbesondere der gerade noch aufgelösten (und somit aus den Röntgenbilddaten auflösbaren) Strukturen dar.

Besonders bevorzugt werden die Hochfrequenzbilddaten mittels eines Hochpassfilters aus den Röntgenbilddaten extrahiert.

Optional werden die Röntgenbilddaten hierzu zunächst vom Ortsraum in den Frequenzraum überführt, bspw. mittels einer Fourier-Transformation, und anschließend mittels des Hochpassfilters gefiltert. Weiter optional erfolgt anschließend auch eine Rücküberführung (Rücktransformation) in den Ortsraum.

Die Röntgenbilddaten sind insbesondere durch pixelweise dargestellte Intensitäten der einfallenden Röntgenstrahlung gebildet oder aus derartigen - insbesondere mittels einzelner Detektorpixel eines Detektors des Röntgengeräts erfassten - Intensitäten hergeleitet. Jedem Pixel ist (oder kann) dabei ein korrespondierendes Signal (werden), im Fall von Detektorpixeln insbesondere ein entsprechendes Mess-Signal zugeordnet. Dieses Signal ist dabei charakteristisch für die jeweilige Intensität und steht dabei konkret für den, die Intensität beziffernden Intensitätswert (auch: "Grauwert"). D. h. das Signal gibt eine quantitative Information über die Größe der jeweiligen Intensität an, unmittelbar (also den jeweiligen Grauwert) oder mittelbar. Bspw. ist das Signal direkt oder indirekt proportional zu dem spezifischen Grauwert, oder steht mit diesem in einer nicht-linearen, beispielsweise einer logarithmischen, exponentiellen oder polynomialen Beziehung.

Bei den aus den korrigierten Hochfrequenzbilddaten erstellten (korrigierten) Bilddaten handelt es sich insbesondere um ein Röntgenbild, das aus den korrigierten Hochfrequenzbilddaten sowie ggf. zusätzlich aus den ursprünglichen Röntgenbilddaten und/oder den extrahierten Hochfrequenzbilddaten erstellt wird. Optional handelt es sich bei diesem Röntgenbild also beispielsweise um ein alle Bildinformationen umfassendes Röntgenbild oder um ein Hochfrequenzbild, das nur hochfrequente (also detailreiche, hochaufgelöste) Bildinformationen enthält. Vorzugsweise stellen die korrigierten Bilddaten dabei (rekonstruierte) Röntgen-Schnittbilder dar.

Dadurch, dass die extrahierten Hochfrequenzbilddaten nichtlinear korrigiert werden, kann der Einfluss von Hochfrequenz-Artefakten, die häufig lokal unterschiedlich auftreten und insbesondere von der Form und Größe der jeweiligen, stark absorbierenden Struktur beeinflusst werden, vorteilhafterweise auch unterschiedlich berücksichtigt und somit ausgeglichen werden. Insbesondere kann dadurch eine artefaktabhängige Korrektur durchgeführt werden, die insbesondere nicht "global" über den gesamten Erfassungsbereich gleichbleibende Auswirkungen aufweist und somit unter Umständen zu einer "Überkorrektur" von Bereichen ohne Artefakte führt. Dadurch lassen sich die Bildqualität und gegebenenfalls der Informationsgehalt der korrigierten Bilddaten erhöhen.

Vorzugsweise werden die Röntgenbilddaten mittels des Röntgengeräts erzeugt und zur weiteren Verarbeitung, insbesondere zur vorstehend beschriebenen Korrektur bereitgestellt.

In einer bevorzugten Verfahrensvariante werden die extrahierten Hochfrequenzbilddaten im Rahmen der nichtlinearen Korrektur insbesondere in Abhängigkeit von ihrer Intensität unterschiedlich korrigiert. Insbesondere erfolgt dabei die Korrektur in Abhängigkeit von der Signalstärke des dem jeweiligen Pixel zugeordneten Signals - bspw. also von dem von dem jeweiligen Detektorpixel ausgegebenen Mess-Signal. Dadurch wird vorteilhafterweise ermöglicht, dass Bildbereiche, in denen sich Hochfrequenz-Artefakte besonders stark - d. h. insbesondere mit einem besonders hohen Intensitäts- oder Grauwert - auswirken auch entsprechend stark korrigiert werden, wohingegen Bereiche mit keinen oder nur geringen Auswirkungen von Artefakten nicht bzw. entsprechend geringer korrigiert werden. Insbesondere wird also die Korrektur an die lokal vorliegende "Stärke" (d. h. insbesondere an die erfasste Strahlungsintensität im Bereich) des Artefakts adaptiert.

In einer weiteren bevorzugten Verfahrensvariante werden die extrahierten Hochfrequenzbilddaten im Rahmen der nichtlinearen Korrektur unterschiedlich skaliert. Es erfolgt somit insbesondere keine "Beschneidung" der jeweiligen Grauwerte der extrahierten Hochfrequenzbilddaten ab einem gegebenenfalls vorgegebenen Schwellwert - wodurch regelmäßig Informationen verloren gehen -, sondern vielmehr eine lokal unterschiedlich starke Abbildung der vorliegenden Grauwerte der extrahierten Hochfrequenzbilddaten auf "abgeleitete" oder resultierende, korrigierte Grauwerte. Dadurch wird in vorteilhafter Weise erreicht, dass die ursprünglichen Informationen - wenn auch mit geringeren Ausprägungen - in der resultierenden Intensitätsverteilung erhalten bleiben.

In einer zweckmäßigen Verfahrensvariante wird im Rahmen der nichtlinearen Korrektur vorzugsweise nur teilweise, nämlich insbesondere in einem nullpunktnahen Teilbereich der extrahierten Hochfrequenzbilddaten eine proportionale (und somit lineare) Skalierung der extrahierten Hochfrequenzbilddaten durchgeführt. Vorzugsweise wird dabei ein Proportionalitätsfaktor von Eins angewendet. In letzterem Fall erfolgt somit in diesem Teilbereich effektiv keine Korrektur und insbesondere keine Skalierung der Hochfrequenzbilddaten. Unter "nullpunktnaher Teilbereich" wird hier und im Folgenden insbesondere ein von Null ausgehender (Werte-)Bereich der in den Hochfrequenzbilddaten enthaltenen Intensität (d. h. insbesondere Grauwerte größer oder gleich Null bis zu einem vorgegebenen Grenzwert (-Betrag) von bspw. 0,02 bis 0,1, insbesondere um etwa 0,05) verstanden. Dadurch wird vorteilhafterweise ermöglicht, dass erwünschte Informationen, die in diesem Teilbereich regelmäßig enthalten sind und beispielsweise für eine Auswertung von Röntgenbildern relevant sind, nicht oder zumindest nur linearer beeinflusst werden.

In einer bevorzugten Weiterbildung wird im Rahmen der nichtlinearen Korrektur in einem an den vorstehend beschriebenen nullpunktnahen Teilbereich anschließenden (zweiten) Teilbereich der extrahierten Hochfrequenzbilddaten eine insbesondere überproportionale Skalierung durchgeführt. D. h. die Korrektur (die Skalierung) nimmt umso mehr zu, je stärker das jeweilige Signal - insbesondere je höher der Betrag (Grauwert) der jeweiligen, einem spezifischen Pixel zugeordneten Intensität - ist. Dadurch wird vorteilhafterweise erreicht, dass in einem Wertebereich "normaler" Intensität - nämlich im nullpunktnahen Teilbereich -, in dem erwartungsgemäß keine artefaktbedingten Auswirkungen vorliegen, keine oder zumindest nur eine geringfügige (lineare) Korrektur und in dem daran anschließenden Wertebereich eine immer stärker werdende Korrektur durchgeführt wird. Die beiden Wertebereiche sind dabei vorzugsweise derart gewählt, dass artefaktbedingte Intensitätswerte (insbesondere mit hoher Wahrscheinlichkeit nur) innerhalb des zweiten Wertebereichs liegen.

In einer zweckmäßigen Verfahrensvariante wird im Rahmen der nichtlinearen Korrektur, insbesondere zur vorstehend beschriebenen überproportionalen Skalierung in dem zweiten Teilbereich (zweiten Wertebereich) eine Arkustangensfunktion herangezogen. Vorzugsweise wird diese Arkustangensfunktion dabei derart parametriert, dass deren "Ursprung" auf das "nullpunktferne Ende" des ersten Wertebereichs gesetzt wird. Vorzugsweise wird diese Arkustangensfunktion dabei im Rahmen einer Skalierungsfunktion, die bereichsweise (nämlich für den ersten und den zweiten Wertebereich) unterschiedlich definiert ist, eingesetzt. Beispielsweise ist die Skalierungsfunktion definiert durch:

$$f(x) = \begin{cases} x & wenn\ 0 \leq x \leq t \\ a \cdot \tan^{-1}\left(\frac{x-t}{a}\right) + t & wenn\ x > t \end{cases} \tag{1}$$

wobei x die pixelspezifische (ggf. gemessene) Intensität und a und t jeweils Parameter zur Anpassung der Arkustangensfunktion hinsichtlich ihrer Skalierungseigenschaften darstellen.

In einer weiteren zweckmäßigen Verfahrensvariante werden vor der Erstellung der korrigierten Bilddaten die korrigierten Hochfrequenzbilddaten insbesondere gewichtet mit den extrahierten Hochfrequenzbilddaten kombiniert. Insbesondere werden die korrigierten Hochfrequenzbilddaten dabei unter Berücksichtigung einer Gewichtungsmaske mit den extrahierten Hochfrequenzbilddaten addiert. Die Gewichtung bzw. die Gewichtungsmaske ist dabei derart gewählt, dass Bildbereiche, die außerhalb von insbesondere metallischen Strukturen oder zumindest außerhalb eines Einflussbereich von durch diese metallischen Strukturen bedingten Artefakten liegen, nicht von den korrigierten Hochfrequenzbilddaten beeinflusst werden. Insbesondere soll dadurch ermöglicht werden, dass die insbesondere in diesen Bildbereichen liegenden und als "korrekt" anzunehmenden Hochfrequenzinhalte nicht fälschlicherweise korrigiert werden. Insbesondere kann dadurch verhindert werden, dass vergleichsweise hohe Intensitäten innerhalb der Hochfrequenzbilddaten, die von der nichtlinearen Korrektur, insbesondere der vorstehend beschriebenen überproportionalen Skalierung erfasst werden, aber außerhalb von durch metallische Strukturen beeinflussten Bildbereichen liegen, korrigiert werden. Insbesondere wird hierzu der Einfluss der Korrektur für derartige Bildbereiche durch die in den extrahierten Hochfrequenzbilddaten enthaltenen Informationen insbesondere nachträglich ausgeglichen. Beispielsweise wird die Gewichtungsmaske dabei auf Basis einer bekannten Stelle einer metallischen Struktur im Körper eines Patienten erstellt.

In einer optionalen Verfahrensvariante werden die extrahierten Hochfrequenzbilddaten vor der nichtlinearen Korrektur derart maskiert, dass die nichtlineare Korrektur nicht auf die vorstehend beschriebenen als korrekt anzunehmenden Hochfrequenzinhalte sondern "nur noch" (d. h. insbesondere ausschließlich) auf die mit hoher Wahrscheinlichkeit durch Hochfrequenz-Artefakte beeinflussten Bildbereiche wirkt. Die Maskierung erfolgt dabei insbesondere analog zu der vorstehend beschriebenen Gewichtungsmaske.

In einer zweckmäßigen Verfahrensvariante werden die Röntgenbilddaten in Form von sogenannten Projektionsbilddaten (auch als "Rohbilddaten" bezeichnet) bereitgestellt. Diese Projektionsbilddaten werden insbesondere in dem Fall, dass es sich bei dem Röntgengerät um ein Computertomographiegerät handelt, durch die mittels der einzelnen Detektorzeilen erfassten Intensitätsverläufe gebildet (auch als "Sinogramm" bezeichnet).

In einer alternativen Verfahrensvariante werden die Röntgenbilddaten bereits in Form von rekonstruierten Bilddaten - d. h. insbesondere in Form von Schnittbildern - bereitgestellt. Optional wird das vorstehend beschriebene Verfahren dabei auf den rekonstruierten Bilddaten durchgeführt.

In einer optionalen Weiterbildung werden die rekonstruierten Bilddaten aber vor der Extraktion der Hochfrequenzbilddaten in ("berechnete") Projektionsbilddaten transformiert. Insbesondere werden hierfür die rekonstruierten Bilddaten mittels einer "Vorwärts-Projektion" in diese Projektionsbilddaten "zurückgerechnet". Dieses Vorgehen kann beispielsweise für den Fall zweckmäßig sein, dass das vorstehend beschriebene Verfahren unabhängig von einem Röntgengerät durchgeführt wird und lediglich bereits rekonstruierte Bilddaten zur Verfügung stehen.

In einer optionalen Verfahrensvariante wird das vorstehend beschriebene Bilderzeugungsverfahren vollautomatisch - d. h. insbesondere ohne Interaktion bspw. mit einem Bediener des Röntgengeräts - durchgeführt.

Der erfindungsgemäße Bilderzeugungsrechner ist dazu eingerichtet, das vorstehend beschriebene Verfahren insbesondere automatisch durchzuführen. Der Bilderzeugungsrechner ist dabei vorzugsweise durch einen Mikrocontroller gebildet, in dem die Funktionalität zur Durchführung des erfindungsgemäßen Bilderzeugungsverfahrens in Form eines Softwaremoduls implementiert ist. Der Bilderzeugungsrechner ist also dazu eingerichtet, aus den bereitgestellten Röntgenbilddaten Hochfrequenzbilddaten zu extrahieren, die extrahierten Hochfrequenzbilddaten anschließend der nichtlinearen Korrektur zu unterziehen und mittels der korrigierten Hochfrequenzbilddaten die korrigierten Bilddaten zu erstellen.

Das erfindungsgemäße Röntgengerät umfasst insbesondere den vorstehend beschriebenen Bilderzeugungsrechner, der dazu eingerichtet ist, das vorstehend beschriebene Verfahren insbesondere automatisch durchzuführen.

Der Bilderzeugungsrechner sowie das Röntgengerät weisen somit die sich aus dem vorstehend beschriebenen Verfahren ergebenden Merkmale und Vorteile in gleicher Weise auf.

Das erfindungsgemäße Computerprogrammprodukt (insbesondere das vorstehend beschriebene Softwaremodul) weist Programmcode (d. h. insbesondere entsprechende Instruktionen) zur Durchführung des vorstehend beschriebenen Verfahrens auf, wenn das Computerprogrammprodukt auf einem Computer (insbesondere auf dem vorstehen beschriebenen Bilderzeugungsrechner) ausgeführt wird.

Das erfindungsgemäße Computerprogrammprodukt ist dabei auf dem erfindungsgemäßen Datenträger gespeichert und somit von diesem umfasst.

Nachfolgend werden Ausführungsbeispiele der Erfindung anhand einer Zeichnung näher erläutert. Darin zeigen:

FIG 1       in einer schematischen Frontalansicht ein Röntgengerät,

FIG 2       in einem schematischen Ablaufdiagramm ein Bilderzeugungsverfahren, das mit dem Röntgengerät durchgeführt wird,

FIG 3       in einem Diagramm schematisch eine Kurve einer Korrekturfunktion für Bildwerte eines mittels des Röntgengeräts erfassten Röntgenbild-Datensatzes,

FIG 4       in Ansicht gemäß FIG 2 ein weiteres Ausführungsbeispiel des Bilderzeugungsverfahrens,

FIG 5       in schematischer Darstellung ein Röntgen-Schnittbild mit Metallartefakten,

FIG 6       in schematischer Darstellung die dem RöntgenSchnittbild gemäß FIG 5 zugeordneten Projektionsbilddaten,

FIG 7       in einem schematischen Diagramm einen Verlauf von in Detektorzeilenrichtung erfasste Grauwerten der Projektionsbilddaten gemäß FIG 6 entlang einer Schnittlinie VII-VII,

FIG 8       in Ansicht gemäß FIG 7 den Verlauf der Grauwerte nach einer Korrektur mittels der Korrekturfunktion gemäß FIG 3,

FIG 9       in Ansicht gemäß FIG 6 die Projektionsdaten nach der Korrektur, und

FIG 10      in Ansicht gemäß FIG 5 das aus den Projektionsbilddaten gemäß FIG 9 abgeleitete Röntgen-Schnittbild.

Einander entsprechende Teile und Größen sind in allen Figuren stets mit gleichen Bezugszeichen versehen.

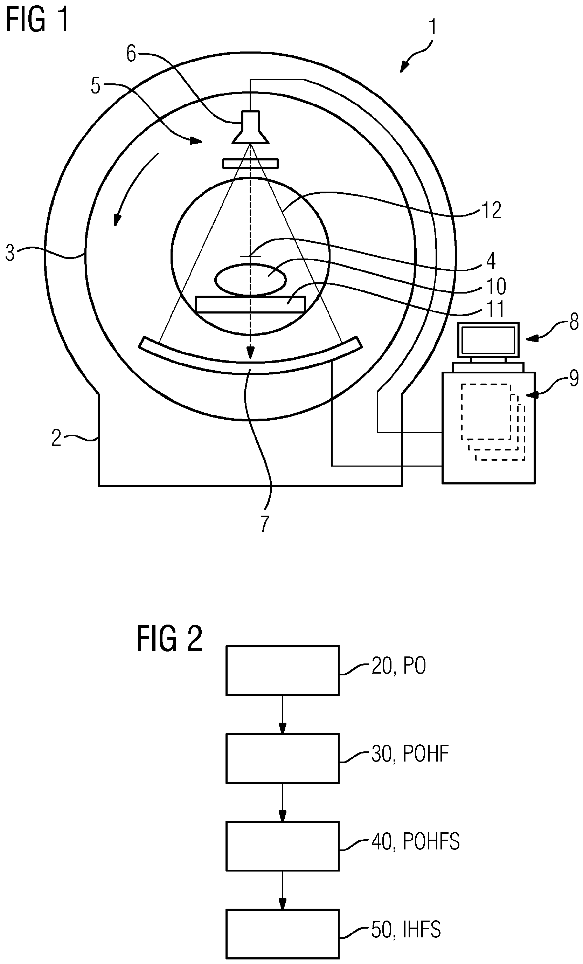

In FIG 1 ist ein Röntgengerät 1 dargestellt, das konkret durch ein Computertomographiegerät gebildet ist. Das Röntgengerät 1 umfasst einen Geräterahmen 2 sowie einen als "Gantry 3" bezeichneten, ringförmigen Drehkranz, der um eine Drehachse 4 (auch als "Isozentrum" bezeichnet) drehbar an dem Geräterahmen 2 gehaltert ist. Die Gantry 3 trägt dabei ein Röntgensystem 5, das durch eine Röntgenstrahlenquelle (kurz als "Röntgenquelle 6" bezeichnet) und einen dazu in Gegenüberstellung gehalterten Röntgenstrahlendetektor (kurz als "Detektor 7" bezeichnet) gebildet ist. Zur Steuerung und Auswertung ist das Röntgensystem 5, konkret die Röntgenquelle 6 und der Detektor 7 mit einem (durch einen Computer gebildeten) Bilderzeugungsrechner 8 signalübertragungstechnisch verbunden. Auf diesem Bilderzeugungsrechner 8 ist ein Bilderzeugungsprogramm 9 (das ein Computerprogrammprodukt darstellt) lauffähig installiert, wodurch der Bilderzeugungsrechner 8 - konkret bei Ausführung des Bilderzeugungsprogramms 9 - dazu eingerichtet ist, ein im Nachfolgenden näher beschriebenes Bilderzeugungsverfahren durchzuführen.

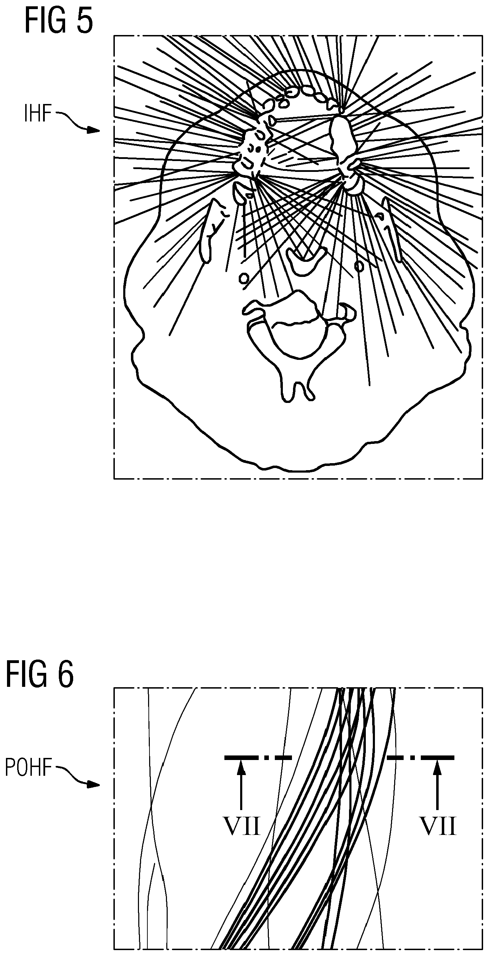

Zur bildgebenden Untersuchung eines Patienten 10 wird dieser im bestimmungsgemäßen Betrieb des Röntgengeräts 1 auf einem Patiententisch 11 des Röntgengeräts 1 abgelegt und auf diesem in die Gantry 3, konkret in einen von dieser gebildeten Untersuchungstunnel verfahren. Dabei rotiert die Gantry 3 um den Patienten 10, während die Röntgenquelle 6 einen Fächerstrahl 12 zur Durchleuchtung des Patienten 10 emittiert. Mittels des Detektors 7, konkret mittels über die Detektorfläche des Detektors 7 rasterartig (konkret in mehreren nebeneinander angeordneten Detektorzeilen) verteilter Detektorpixel (nicht näher dargestellt), wird dabei die einfallende Röntgenstrahlung detektiert und in Form einer Intensitätsverteilung, die die lokale Abschwächung des Fächerstrahls 12 repräsentiert, an den Bilderzeugungsrechner 8 ausgegeben. Aufgrund der Rotation der Gantry 3 und damit des Detektors 7 sowie aufgrund der Anordnung der Pixel des Detektors 7 in den etwa tangential zur Rotationsachse 4 stehenden Detektorzeilen (sowie aufgrund der Vorschubbewegung des Patiententischs 11 entlang der Rotationsachse 4) ergeben die mittels des Detektors 7 erfassten Rohdaten (als "Projektionsbilddaten PO" bezeichnet) ein Bild, das etwa dem in FIG 6 dargestellten Bild entspricht (auch als "Sinogramm" bezeichnet). Zur Erzeugung des Röntgen-Schnittbilds, das eine im Wesentlichen normal zur Rotationsachse 4 stehende Schnittebene durch den Patienten 10 darstellt, werden diese Projektionsbilddaten PO mittels einer sogenannten Rück-Projektion in eine "Bild-Domäne" überführt und dabei in Bilddaten I0 transformiert.

Für den Fall, dass im Körper des Patienten 10 metallische Teile vorhanden sind, beispielsweise ein Implantat, kann es zu unerwünschten Metall-Artefakten kommen, die sich in den Projektionsbilddaten PO unter anderem aufgrund der oft scharf abgegrenzten Übergänge zwischen stark unterschiedlichen Röntgenabsorptionswerten an den Kanten der metallischen Teile als strahlenartige Bildfehler mit teilweise hohen Grauwerten äußern, wie sie beispielsweise in FIG 5 im oberen Bildbereich zu erkennen sind. Diese Bildfehler gleichen dabei vergleichsweise kleinen Strukturen, bspw. Kanten, die aber in Realität nicht vorhanden sind. Der Bilderzeugungsrechner 8 ist mittels des Bilderzeugungsprogramms 9 dazu eingerichtet, bei Durchführung des Bilderzeugungsverfahrens derartige Hochfrequenz-Artefakte zu verringern.

In FIG 2 ist ein erstes Ausführungsbeispiel des Bilderzeugungsverfahrens näher dargestellt. In einem ersten Verfahrensschritt 20 werden von dem Bilderzeugungsrechner 8 an das Bilderzeugungsprogramm 9 die mittels des Detektors 7 erfassten Projektionsbilddaten PO als Röntgenbilddaten bereitgestellt.

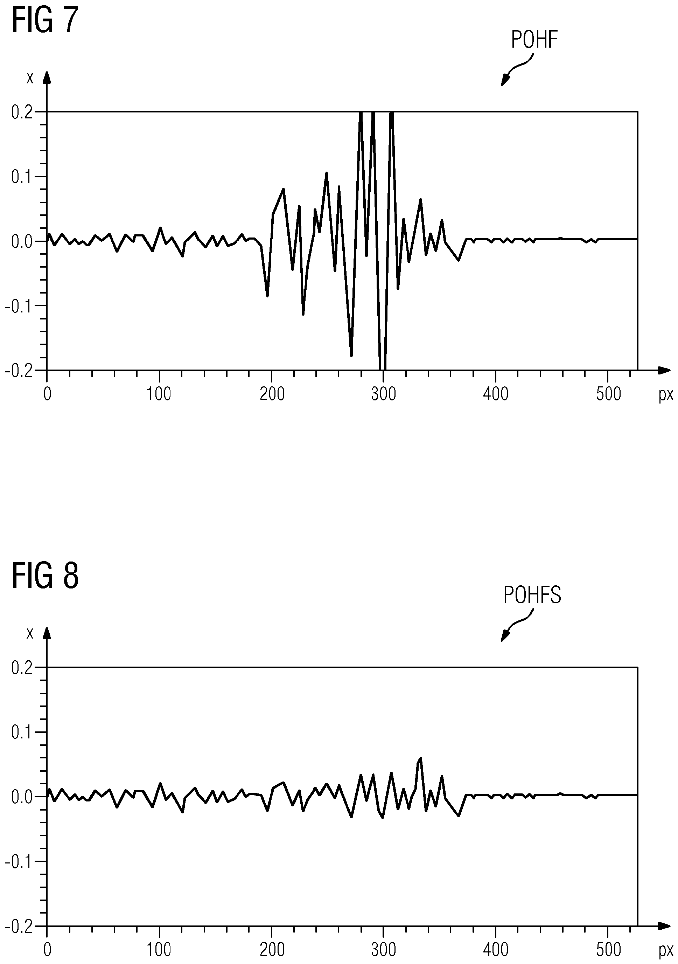

In einem darauffolgenden Verfahrensschritt 30 werden aus den Projektionsbilddaten PO Hochfrequenzbilddaten POHF extrahiert. Konkret wird dabei auf die Projektionsbilddaten PO ein Hochpassfilter angewendet. Dadurch sind in den Hochfrequenzbilddaten POHF "nur noch" hochfrequente Informationen, d. h. auf besonders kleine Strukturen bezogene Informationen, konkret eine damit verknüpfte Intensitätsverteilung enthalten. Die Hochfrequenzbilddaten POHF sind in FIG 6 dargestellt. Wie aus FIG 7 entnommen werden kann, in der ein örtlicher Verlauf der erfassten Intensität x über die einzelnen Pixel in Detektorzeilenrichtung, konkret entlang einer Schnittlinie VII-VII nach FIG 6 dargestellt ist, weisen an dieser Stelle die Hochfrequenzbilddaten POHF im Bereich zwischen den Pixelnummern 180-380 signifikant erhöhte Intensitäten x (d. h. jeweils stark erhöhte Grauwerte) auf. Diese erhöhten Grauwerte stellen einen anomalen Verlauf der Intensität x in Detektorzeilenrichtung dar und lassen auf metallbedingte Hochfrequenz-Artefakte schließen. Diese Artefakte führen wiederum bei Rekonstruktion des Hochfrequenz-Schnittbilds IHF aus den Hochfrequenzbilddaten POHF (dargestellt in FIG 5) zu den dort ersichtlichen strahlenartigen Effekten.

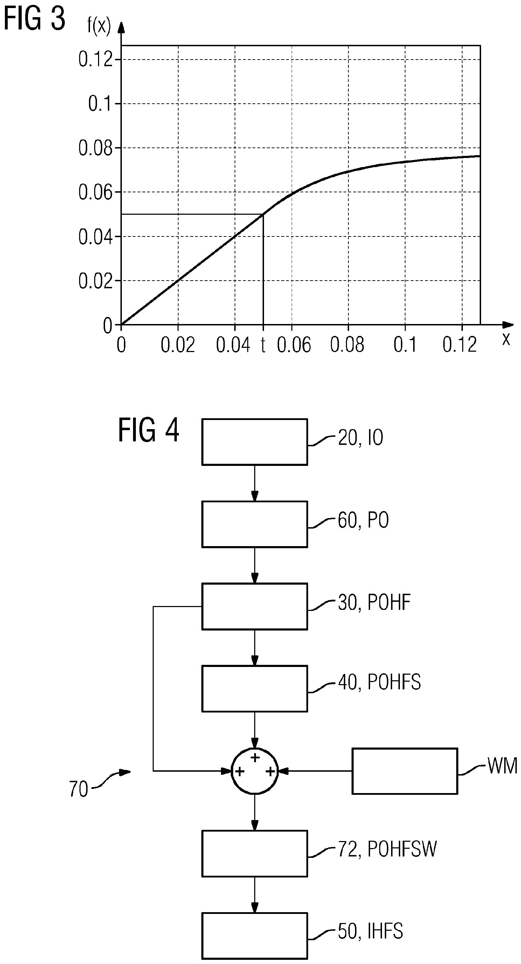

In einem nachfolgenden Verfahrensschritt 40 werden die extrahierten Hochfrequenzbilddaten POHF einer Korrektur, konkret einer nichtlinearen Skalierung unterworfen. Die entsprechende Skalierungsfunktion f(x), konkret deren Verlauf über die zugeordnete Intensität x eines einzelnen Pixels ist in FIG 3 näher dargestellt. Die Skalierungsfunktion f(x) ist dabei derart parametriert und bereichsweise unterschiedlich definiert, dass sie in einem Grauwerte-Bereich zwischen 0 und 0,05 durch eine proportionale Abbildungsfunktion mit einem Proportionalitätsfaktor von 1 gebildet ist. D. h. im Bereich zwischen 0 und 0,05 erfolgt keine Skalierung der Grauwerte. In einem daran anschließenden zweiten Wertebereich - d. h. für Grauwerte der Intensität x größer 0,05 - ist die Skalierungsfunktion f(x) durch eine Arkustangensfunktion gebildet. Die Skalierungsfunktion f(x) hat dabei konkret die Form:

$$f(x) = \begin{cases} x & wenn\ 0 \leq x \leq t \\ a \cdot \tan^{-1}\left(\frac{x-t}{a}\right) + t & wenn\ x > t \end{cases},$$

wobei im vorliegenden Ausführungsbeispiel t den Betrag 0,05 und a den Betrag 0,02 aufweisen.

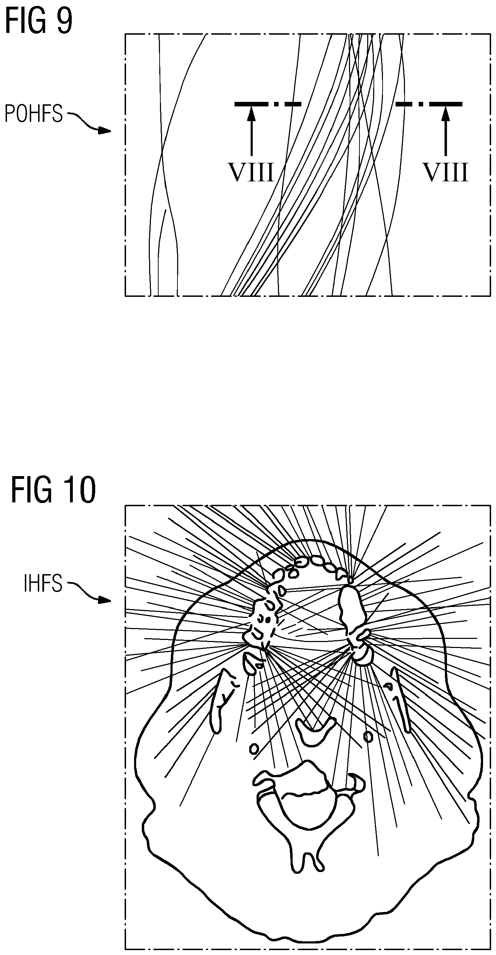

Diese Skalierungsfunktion f(x) wird nun im Verfahrensschritt 40 auf die Hochfrequenzbilddaten POHF angewendet und diese somit in korrigierte oder skalierte Hochfrequenzbilddaten POHFS überführt. Konkret werden dabei die Intensitäten x, deren Grauwertbeträge größer als 0,05 sind, überproportional zunehmend skaliert. Das Ergebnis ist in FIG 8 und 9 dargestellt.

In einem nachfolgenden Verfahrensschritt 50 werden aus den skalierten Hochfrequenzbilddaten POHFS korrigierte (oder: skalierte) Bilddaten IHFS erstellt. Im vorliegenden Ausführungsbeispiel gemäß FIG 2 werden dazu die skalierten Hochfrequenzbilddaten POHFS mittels Rück-Projektion in die korrigierten Bilddaten IHFS transformiert. Das aus den korrigierten Bilddaten IHFS abgeleitete Schnittbild ist in FIG 10 dargestellt.

In FIG 4 ist ein weitergebildetes Ausführungsbeispiel des Bilderzeugungsverfahrens näher dargestellt. In diesem Ausführungsbeispiel werden im ersten Verfahrensschritt 20 als Röntgenbilddaten rekonstruierte Röntgenbilddaten I0 herangezogen. Diese werden in einem Verfahrensteilschritt 60 des Verfahrensschritts 20 in die vorstehend beschriebenen Projektionsdaten PO mittels Vorwärts-Projektion transformiert. Die nachfolgenden Verfahrensschritte 30 und 40 entsprechen den vorstehend im Rahmen des Ausführungsbeispiels gemäß FIG 2 beschriebenen Verfahrensschritten 30 und 40.

Um jedoch den Einfluss der nichtlinearen Skalierungsfunktion f(x) in Bildbereichen, in denen die zugeordnete Intensität x zwar ebenfalls hohe Grauwertbeträge annehmen aber nicht durch metallische Teile am Körper des Patienten 10 verfälscht sein kann, nicht unerwünscht mittels der Skalierungsfunktion f(x) zu korrigieren, werden die im Verfahrensschritt 30 extrahierten Hochfrequenzbilddaten POHF in einem weiteren Verfahrensschritt 70 mit den skalierten Hochfrequenzbilddaten POHFS gewichtet kombiniert. Zur (lokalen) Gewichtung wird dazu im Verfahrensschritt 70 eine Metall-Gewichtungsmaske WM herangezogen. Diese Metall-Gewichtungsmaske WM lässt konkret nur dort eine Änderung der Hochfrequenzbilddaten POHF zu, wo mit hoher Wahrscheinlichkeit ein Einfluss eines metallischen Teils zu erwarten ist. Aus dieser Kombination ergeben sich in einem Verfahrenszwischenschritt 72 gewichtete, skalierte Hochfrequenzbilddaten POHFSW.

In dem nachfolgenden Verfahrensschritt 50 werden die gewichteten, skalierten Hochfrequenzbilddaten POHFSW analog zum Ausführungsbeispiel gemäß FIG 2 mittels Rück-Projektion in die korrigierten Bilddaten IHFS transformiert.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

**Patentansprüche**

1. Verfahren zur Bilderzeugung bei einem Röntgengerät (1), wobei verfahrensgemäß

   - Röntgenbilddaten (I0,P0) bereitgestellt werden,
   - aus den Röntgenbilddaten (I0,P0) Hochfrequenzbilddaten (POHF), die eine Hochfrequenzinformation enthalten, extrahiert werden,
   - die extrahierten Hochfrequenzbilddaten (POHF) einer nichtlinearen Korrektur unterworfen werden, und
   - wobei mittels der korrigierten Hochfrequenzbilddaten (POHFS,POHFSW) korrigierte Bilddaten (IHFS) erstellt werden.

2. Verfahren nach Anspruch 1,
   wobei die extrahierten Hochfrequenzbilddaten (POHF) im Rahmen der nichtlinearen Korrektur in Abhängigkeit von ihrer Intensität (x) unterschiedlich korrigiert werden.

3. Verfahren nach Anspruch 1 oder 2,
   wobei die extrahierten Hochfrequenzbilddaten (POHF) im Rahmen der nichtlinearen Korrektur unterschiedlich skaliert werden.

4. Verfahren nach einem der Ansprüche 1 bis 3,
   wobei im Rahmen der nichtlinearen Korrektur in einem nullpunktnahen Teilbereich der extrahierten Hochfrequenzbilddaten (POHF) eine proportionale Skalierung der extrahierten Hochfrequenzbilddaten (POHF), insbesondere mit einem Proportionalitätsfaktor von 1, durchgeführt wird.

5. Verfahren nach Anspruch 4,
   wobei im Rahmen der nichtlinearen Korrektur in einem an den nullpunktnahen Teilbereich anschließenden Teilbereich der extrahierten Hochfrequenzbilddaten (POHF) eine überproportionale Skalierung durchgeführt wird.

6. Verfahren nach einem der Ansprüche 1 bis 5,
   wobei im Rahmen der nichtlinearen Korrektur, insbesondere zur überproportionalen Skalierung, eine Arkustangensfunktion herangezogen wird.

7. Verfahren nach einem der Ansprüche 1 bis 6,
   wobei vor der Erstellung der korrigierten Röntgenbilddaten (IHF) die korrigierten Hochfrequenzbilddaten (POHFS) gewichtet mit den extrahierten Hochfrequenzbilddaten (POHF) kombiniert werden.

8. Verfahren nach einem der Ansprüche 1 bis 7,
   wobei die Röntgenbilddaten in Form von Projektionsbilddaten (PO) bereitgestellt werden.

9. Verfahren nach einem der Ansprüche 1 bis 7,
   wobei die Röntgenbilddaten in Form von rekonstruierten Bilddaten (I0) bereitgestellt werden.

10. Verfahren nach Anspruch 9,
    wobei die rekonstruierten Bilddaten (I0) vor der Extraktion der Hochfrequenzbilddaten (POHF) in berechnete Projektionsbilddaten (PO) transformiert werden.

**11.** Bilderzeugungsrechner (8), der dazu eingerichtet ist, das Verfahren nach einem der Ansprüche 1 bis 10 durchzuführen.

**12.** Röntgengerät (1) mit einem Bilderzeugungsrechner (8), der dazu eingerichtet ist, das Verfahren nach einem der Ansprüche 1 bis 10 durchzuführen.

**13.** Computerprogrammprodukt (9) mit Programmcode zur Durchführung des Verfahrens zur Bilderzeugung nach einem der Ansprüche 1 bis 10, wenn das Computerprogrammprodukt auf einem Computer (8) ausgeführt wird.

**14.** Computerlesbarer Datenträger, der das Computerprogrammprodukt (9) nach Anspruch 13 umfasst.

**Geänderte Patentansprüche gemäss Regel 137(2) EPÜ.**

**1.** Verfahren zur Bilderzeugung bei einem Röntgengerät (1), wobei verfahrensgemäß

- Röntgenbilddaten (I0,P0) bereitgestellt werden,
- aus den Röntgenbilddaten (I0,P0) Hochfrequenzbilddaten (P0HF), die eine Hochfrequenzinformation enthalten, extrahiert werden,
- die extrahierten Hochfrequenzbilddaten (P0HF) einer nichtlinearen Korrektur unterworfen werden, und
- wobei mittels der korrigierten Hochfrequenzbilddaten (P0HFS,P0HFSW) korrigierte Bilddaten (IHFS) erstellt werden

**dadurch gekennzeichnet, dass**
im Rahmen der nichtlinearen Korrektur in einem nullpunktnahen Teilbereich der extrahierten Hochfrequenzbilddaten (P0HF) eine proportionale Skalierung der extrahierten Hochfrequenzbilddaten (P0HF) durchgeführt wird,
und dass
im Rahmen der nichtlinearen Korrektur in einem an den nullpunktnahen Teilbereich anschließenden Teilbereich der extrahierten Hochfrequenzbilddaten (P0HF) eine überproportionale Skalierung durchgeführt wird.

**2.** Verfahren nach Anspruch 1,
wobei die extrahierten Hochfrequenzbilddaten (POHF) im Rahmen der nichtlinearen Korrektur in Abhängigkeit von ihrer Intensität (x) unterschiedlich korrigiert werden.

**3.** Verfahren nach Anspruch 1 oder 2,
wobei die extrahierten Hochfrequenzbilddaten (POHF) im Rahmen der nichtlinearen Korrektur unterschiedlich skaliert werden.

**4.** Verfahren nach einem der Ansprüche 1 bis 3,
wobei die proportionale Skalierung der extrahierten Hochfrequenzbilddaten (POHF) mit einem Proportionalitätsfaktor von 1 durchgeführt wird.

**5.** Verfahren nach einem der Ansprüche 1 bis 4,
wobei im Rahmen der nichtlinearen Korrektur, insbesondere zur überproportionalen Skalierung, eine Arkustangensfunktion herangezogen wird.

**6.** Verfahren nach einem der Ansprüche 1 bis 5,
wobei vor der Erstellung der korrigierten Röntgenbilddaten (IHF) die korrigierten Hochfrequenzbilddaten (POHFS) gewichtet mit den extrahierten Hochfrequenzbilddaten (P0HF) kombiniert werden.

**7.** Verfahren nach einem der Ansprüche 1 bis 6,
wobei die Röntgenbilddaten in Form von Projektionsbilddaten (P0) bereitgestellt werden.

**8.** Verfahren nach einem der Ansprüche 1 bis 6,
wobei die Röntgenbilddaten in Form von rekonstruierten Bilddaten (I0) bereitgestellt werden.

**9.** Verfahren nach Anspruch 8,
wobei die rekonstruierten Bilddaten (I0) vor der Extraktion der Hochfrequenzbilddaten (P0HF) in berechnete Projektionsbilddaten (P0) transformiert werden.

**10.** Bilderzeugungsrechner (8), der dazu eingerichtet ist, das Verfahren nach einem der Ansprüche 1 bis 9 durchzuführen.

**11.** Röntgengerät (1) mit einem Bilderzeugungsrechner (8), der dazu eingerichtet ist, das Verfahren nach einem der Ansprüche 1 bis 9 durchzuführen.

**12.** Computerprogrammprodukt (9) mit Programmcode zur Durchführung des Verfahrens zur Bilderzeugung nach einem der Ansprüche 1 bis 9, wenn das Computerprogrammprodukt auf einem Computer (8) ausgeführt wird.

**13.** Computerlesbarer Datenträger, der das Computerprogrammprodukt (9) nach Anspruch 12 umfasst.

FIG 1

FIG 2

**FIG 3**

**FIG 4**

## FIG 7

## FIG 8

FIG 9

POHFS

VIII          VIII

FIG 10

IHFS

Europäisches
Patentamt

European
Patent Office

Office européen
des brevets

## EUROPÄISCHER RECHERCHENBERICHT

Nummer der Anmeldung

EP 18 16 8142

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | STAHL MARTIN ET AL: "Digital radiography enhancement by nonlinear multiscale processing", MEDICAL PHYSICS, AIP, MELVILLE, NY, US, Bd. 27, Nr. 1, 1. Januar 2000 (2000-01-01), Seiten 56-65, XP012010959, ISSN: 0094-2405, DOI: 10.1118/1.598857 * das ganze Dokument * ----- | 1-14 | INV. A61B6/00 |
| A | US 9 508 127 B1 (KATSUHARA SHINSUKE [US] ET AL) 29. November 2016 (2016-11-29) * Spalte 2, Zeile 32 - Spalte 14, Zeile 42 * ----- | 1-14 | |
| A | EP 1 713 031 A1 (AGFA GEVAERT [BE]) 18. Oktober 2006 (2006-10-18) * Absatz [0015] - Absatz [0035] * ----- | 1-14 | |
| A | US 2010/239146 A1 (SUZUKI TATSURO [JP]) 23. September 2010 (2010-09-23) * Absatz [0055] - Absatz [0164] * ----- | 1-14 | |
| A,D | DE 10 2016 202434 A1 (SIEMENS HEALTHCARE GMBH [DE]) 17. August 2017 (2017-08-17) * Absatz [0045] - Absatz [0060] * ----- | 1-14 | RECHERCHIERTE SACHGEBIETE (IPC) A61B |
| A,D | DE 10 2014 217966 A1 (SIEMENS AG [DE]) 10. März 2016 (2016-03-10) * Absatz [0025] - Absatz [0038] * ----- | 1-14 | |

Der vorliegende Recherchenbericht wurde für alle Patentansprüche erstellt

| Recherchenort | Abschlußdatum der Recherche | Prüfer |
|---|---|---|
| München | 19. Oktober 2018 | Hooper, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 16 8142

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-10-2018

| Im Recherchenbericht angeführtes Patentdokument | Datum der Veröffentlichung | Mitglied(er) der Patentfamilie | Datum der Veröffentlichung |
|---|---|---|---|
| US 9508127 B1 | 29-11-2016 | JP 2017060737 A<br>US 9508127 B1 | 30-03-2017<br>29-11-2016 |
| EP 1713031 A1 | 18-10-2006 | EP 1713031 A1<br>JP 5008333 B2<br>JP 2006289095 A | 18-10-2006<br>22-08-2012<br>26-10-2006 |
| US 2010239146 A1 | 23-09-2010 | JP 5641748 B2<br>JP 2010240387 A<br>US 2010239146 A1 | 17-12-2014<br>28-10-2010<br>23-09-2010 |
| DE 102016202434 A1 | 17-08-2017 | CN 107093198 A<br>DE 102016202434 A1<br>US 2017236309 A1 | 25-08-2017<br>17-08-2017<br>17-08-2017 |
| DE 102014217966 A1 | 10-03-2016 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016202434 A1
- DE 102014217966 A1